Europäisches Patentamt

European Patent Office

Office européen des brevets

(19)

(11) Veröffentlichungsnummer : **0 134 750**
**B1**

(12)
# EUROPÄISCHE PATENTSCHRIFT

(45) Veröffentlichungstag der Patentschrift :
30.12.86

(21) Anmeldenummer : 84810060.8

(22) Anmeldetag : 02.02.84

(51) Int. Cl.⁴ : **A 61 B 17/28**, A 61 B 17/32

(54) **Parametriumschneidklemme.**

(30) Priorität : 13.09.83 CH 4972/83

(43) Veröffentlichungstag der Anmeldung :
20.03.85 Patentblatt 85/12

(45) Bekanntmachung des Hinweises auf die Patenterteilung : 30.12.86 Patentblatt 86/52

(84) Benannte Vertragsstaaten :
AT BE CH DE FR GB IT LI NL SE

(56) Entgegenhaltungen :
WO-A-83 /009 94
DE-C- 929 750
FR-A- 2 445 133
US-A- 1 918 700

(73) Patentinhaber : FRITZ GEGAUF AG BERNINA-
NAEHMASCHINENFABRIK
Seestrasse
CH-8266 Steckborn (CH)

(72) Erfinder : Lahodny, Johann
Conradstrasse 36
A-3950 Gmünd (AT)
Erfinder : Dreier, Ernst
Mühlhofstrasse 27
CH-8266 Steckborn (CH)

(74) Vertreter : Steiner, Martin et al
c/o AMMANN PATENTANWAELTE AG BERN
Schwarztorstrasse 31
CH-3001 Bern (CH)

EP 0 134 750 B1

## Beschreibung

Die vorliegende Erfindung betrifft eine Parametriumschneidklemme mit Klemmbacken und einer Verriegelungsvorrichtung zum Arretieren der Klemmbacken in Klemmstellung. Bekannte Parametriumklemmen dienen zum Abklemmen von Gewebe, insbesondere des Parametriums zwischen Gebärmutter und Beckenwand bei der operativen Entfernung einer Gebärmutter (DE-GM 80 05 113). Diese bekannten Parametriumklemmen dienen ausschliesslich einem Zweck nämlich dem Abklemmen von Gewebe. Für den Trennvorgang muss ein zusätzliches Instrument zu Hilfe genommen werden, was nicht nur den Operationsvorgang erschwert und verlängert, sondern auch die Gefahr in sich birgt, dass über die geklemmte Partie hinaus geschnitten wird.

Es ist zwar eine chirurgische Zange zum Abklemmen und nachträglichen Durchschneiden von Gefässen bekannt (US-PS 3 175 556). Diese Zange gestattet auf abzutrennende und abzuschliessende Gefässe mittels Klemmbacken nebeneinander zwei Klammern aufzuklemmen und dann mittels einer separat betätigbaren Schneidklinge das Gefäss zwischen den beiden aufgepressten Klammern zu trennen. Die Anwendung dieses bekannten Instrumentes ist jedoch beschränkt auf das Abschliessen und Trennen von Gefässen. Weder das Instrument noch die anzubringenden Klammern sind dazu geeignet in einem fortlaufenden Vorgang Gewebeteile abzuklemmen und anschliessend auf der geklemmten Länge zu trennen.

Es ist weiter eine Zange mit Klemmbacken zum Fassen einer chirurgischen Nadel und mit einer separat betätigbaren Schneidklinge zum Durchschneiden des Nähfadens bekannt (PCT-Anmeldung WO83/00994). Auch diese Zange ist jedoch weder zum Abklemmen von Gewebe noch zum Durchschneiden desselben seitlich der Klemmbacken bestimmt und geeignet.

Ziel vorliegender Erfindung ist es, eine Parametriumschneidklemme zu schaffen, welche durch kombiniertes Klemmen und Schneiden den Operationsvorgang erheblich zu vereinfachen und zu beschleunigen und mit grösserer Sicherheit durchzuführen gestattet, indem nie weiter geschnitten werden kann als abgeklemmt ist. Die Lösung der gestellten Aufgabe ist im Anspruch 1 umschrieben. Eine zusätzliche Erhöhung der Sicherheit und zugleich Vereinfachung der Arbeit kann erzielt werden, wenn die Schnittkante der Schneidklinge bei Offenstellung der Klemmbacken und der Schneidklinge hinter eine Schutzrampe an der einen Klemmbacke greift. Es wird damit verunmöglicht, dass beim Einführen der offenen Klemme zum Erfassen und Klemmen eines Gewebeabschnittes das zu erfassende oder umliegendes Gewebe durch die offene Schneidklinge ungewollt verletzt wird. Vorzugsweise kann hierbei die Schneidklinge durch eine Rastvorrichtung in ihrer offenen, unwirksamen Stellung hinter der Schutzrampe gehalten sein. Damit ist gewährleistet, dass die Schneidklinge nicht zufällig aus ihrer geschützten Ruhestellung verschwenkt werden kann, bevor der Schneidvorgang bewusst ausgelöst werden soll. Es ist hierbei möglich, die Rastkraft derart zu bemessen, dass nach dem Auslösen der Rastvorrichtung geklemmtes Gewebe schlagartig durchschnitten wird.

Die erfindungsgemässe Parametriumschneidklemme wird nun anhand eines in der Zeichnung dargestellten Ausführungsbeispiels näher erläutert.

Figur 1 ist eine Seitenansicht der Parametriumschneidklemme,

Figur 2 ist eine Draufsicht auf die Parametriumschneidklemme,

Figur 3 ist ein Schnitt nach Linie III-III in Fig. 1, in grösserem Massstab und

Figur 4 ist ein Schnitt nach Linie IV-IV in Fig. 1, in grösserem Massstab.

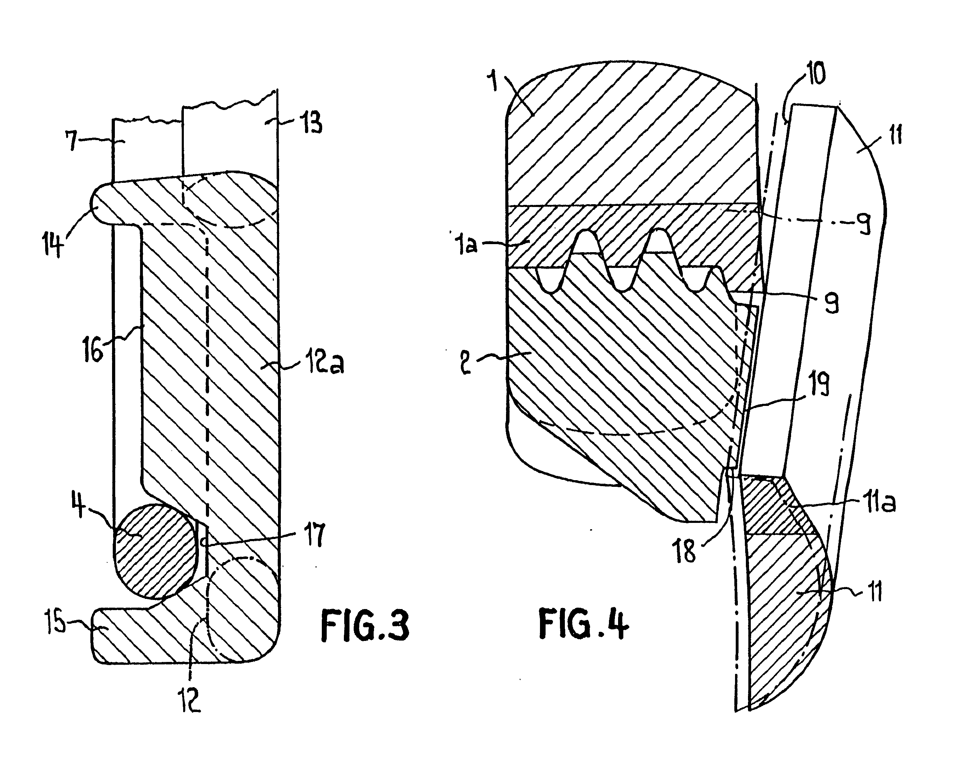

Die dargestellte Parametriumschneidklemme weist eine obere Klemmbacke 1 und eine untere Klemmbacke 2 auf, die mit Branchen 3 bzw. 4 verbunden und um eine Gelenkschraube 5 schwenkbar sind. Die Branchen 3 und 4 sind mit Ringgriffen 6 bzw. 7 versehen, die in üblicher Weise der Betätigung dienen. Querarme der Branchen 3 und 4 sind mit Verriegelungsverzahnungen 8 versehen, welche die Verriegelung der Klemme in verschiedenen geschlossenen Klemmstellungen gestatten. Wie insbesondere Fig. 4 zeigt ist die obere Klemmbacke 1 mit einer Klemmplatte 1a aus Hartmetall belegt. Sowohl diese Klemmplatte 1a als auch die Oberseite der Klemmbacke 2 sind mit Längs- und Querrillen versehen, die insgesamt eine eigentliche Verzahnung bilden und damit die Haftung der Klemme an einem zwischen den Klemmbacken eingeklemmten Gewebe erheblich erhöhen. Die eine seitliche Kante der Klemmplatte 1a ist als Schnittkante 9 längs der einen Seite der Klemmbacke 1 ausgebildet. Sie ist bestimmt zum Zusammenwirken mit einer Schnittkante 10 einer auf eine Schneidklinge 11 aufgesetzten Hartmetallschneide 11a. Die Schneidklinge 11 besteht aus einem Stück mit einer Branche 12, deren hinteres Ende 12a nach aussen abgewinkelt und zu einem Ringgriff 13 erweitert ist. Die Schneidklinge 11 ist ebenfalls auf der Schraube 5 schwenkbar gelagert.

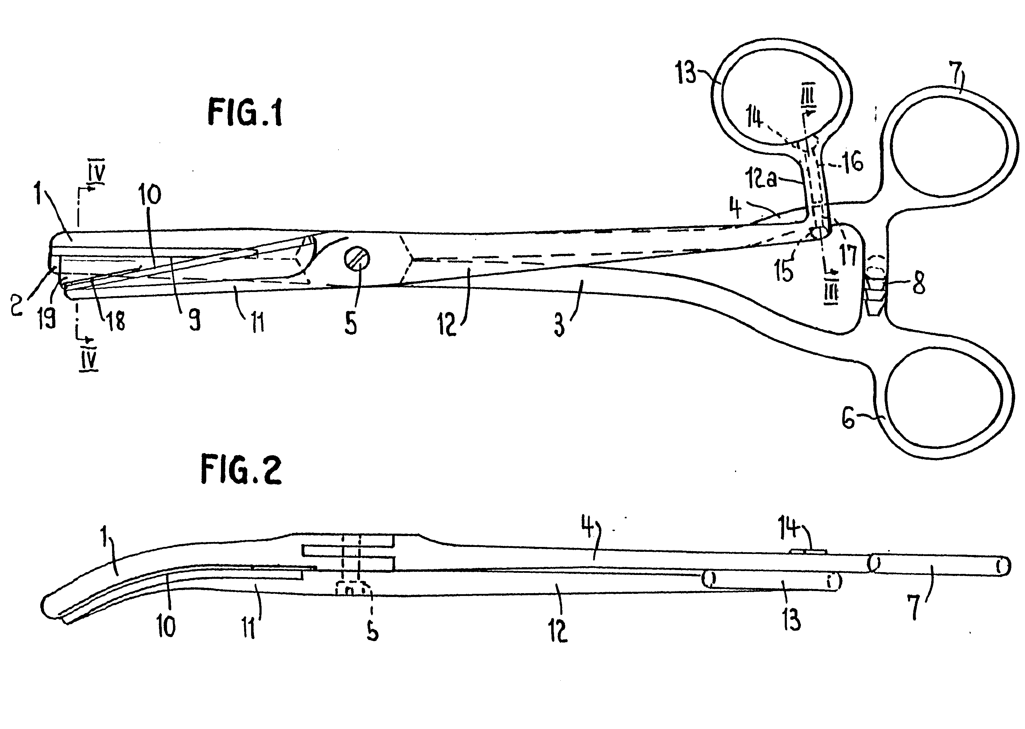

Das auswärts gerichtete hintere Ende der Branche 12 ist gemäss Fig. 3 als Rastnocken ausgebildet, der mit der Branche 4 der unteren Klemmbacke 2 zusammen als Rastvorrichtung wirkt. Der Rastnocken weist zwischen Endanschlägen 14 und 15 einen flachen Teil 16 und am inneren Ende eine Rastvertiefung 17 auf. In dieser Rastvertiefung 17 liegt die Branche 4, bei der in den Fig. 1 und 3 dargestellter unwirksamen Raststellung der Schneidklinge 11. Die Schneidklinge befindet sich bei dieser Raststellung gemäss Fig. 4 etwas unterhalb einer Schulter 18 an der Unterseite einer Schutzrampe 19 der unteren Klemmbacke 2. Wie Fig. 1 zeigt, ist die Schulter 18 bzw. untere Begrenzungsfläche der Schutzrampe 19 entsprechend der Neigung der Schnittkante 10 der Schneidklinge 11 geneigt, so dass diese Schnittkante 10 bei der dargestellten Raststellung der Schneidklinge in gleichmässigem Abstand etwas unterhalb der Schulter 18 liegt. Die Schnittkante 10 der Schneidklinge 11 kann somit unter die Schulter 18 greifen, wie Fig. 4 in strickpunktierten Linien andeutet. Wie Fig. 1 zeigt, ragt die Schulter 18 etwas über die Schnittkante 10 nach vorne, so dass diese Schnittkante bei der strichpunktiert angedeuteten, eingerückten Stellung völlig geschützt und wirkungslos unter der Schutzrampe 19 liegt. Bei der in den Figuren dargestellten, geschlossenen Stellung der Klemme, bei welcher die Rastverzahnungen 8 eingerastet sind, drängt die verhältnismässig weit unten liegende Schnittkante 9 der Klemmplatte 1a der oberen Klemmbacke 1 die Schneidklinge 11 in die ausgezogen dargestellte, äussere Lage, bei der ihre Schnittkante 10 etwas ausserhalb der Schutzrampe 19 liegt. Wird die Klemme noch stärker zusammengedrückt, wird die Schneidklinge 11 noch etwas weiter nach aussen gedrängt. Bei geöffneter Klemme liegt dagegen die Schnittkante 9 der oberen Klemmbacke weiter oben, wie in Fig. 4 in strichpunktierten Linien angedeutet und gibt die Schneidklinge 11 so weit frei, dass ihre Schnittkante 10 unter die Schulter 18 eintreten kann.

Zum Arbeiten mit der Parametriumschneidklemme wird die Schneidklinge in die dargestellte, unwirksame Stellung gebracht, welche durch Einrasten der Branche 4 in die Ausnehmung 17 bestimmt ist. Nun wird die Klemme mittels der Griffe 6 und 7, nötigenfalls unter Ausrücken der Verriegelungsverzahnungen 8 so weit geöffnet, dass sie über das zu erfassende Gewebe, beispielsweise das Parametrium, eingeschoben werden kann. Die Schneidklinge befindet sich bei offener Klemme in der in Fig. 4 in strichpunktierten Linien angedeuteten Lage, d. h., ihre Schnittkante 10 ist abgedeckt und wirkungslos. Es ist daher unmöglich, dass sich die Schneidklinge vorzeitig öffnen und damit Gewebe verletzen oder aber ungewollt schliessen und damit das abzuklemmende und abzuschneidende Gewebe verletzen kann bevor die Klemmung erfolgt ist. Zu diesem Zwecke ist die Rastkraft der Branche 4 in der Rastvertiefung 17 verhältnismässig hoch bemessen. Sind die Klemmbacken 1 und 2 über den zu klemmenden Gewebeabschnitt eingeschoben, wird die Klemme mittels der Griffe 6 und 7 geschlossen und das Gewebe mit der erforderlichen Kraft abgeklemmt, worauf die Klemme durch Einrücken der Verriegelungsverzahnungen 8 in der Klemmlage bleibt. Die Zahnung der Klemmbacken verhindert jedes Verrutschen der Klemme auf dem Gewebe oder umgekehrt des Gewebes in der Klemme. Mit dem Schliessen der Klemmbacken hat die obere Klemmbacke 1 die Schneidklinge 11 aus dem Bereiche der Schulter 18 in die in Fig. 4 ausgezogen dargestellte Lage gebracht, sodass diese Schneidklinge nun die Schnittbewegung durch Verschwenken nach oben ausführen kann. Hierzu wird der Ringgriff 13 nach unten gedrückt, bis die Rastkraft der Rastvorrichtung überwunden ist. Da diese Rastkraft recht hoch gewählt wird, erfolgt nach dem Ausrücken der Rast eine schlagartige Verschwenkung der Schneidklinge und damit ein schlagartiges sauberes Durchschneiden des geklemmten Gewebes. Der Vollständigkeit halber sei erwähnt, dass nach erfolgtem Klemmen das Gewebe auf der der Schneidklinge gegenüberliegenden Seite der Klemmbacken vernäht wird, bevor das soeben erwähnte Durchtrennen durch Betätigung der Schneidklinge erfolgt. Gerade weil dieser zusätzliche Arbeitsgang bei geschlossener Klemme, also aus dem Bereiche der Schulter 18 ausgerückten Schneidklinge erfolgt, ist es besonders wichtig die Betätigung der Schneidklinge nur unter Ueberwindung einer erheblichen Rastkraft zu gestatten, damit nicht beim Nähvorgang die Gefahr einer unbeabsichtigten Betätigung der Schneidklinge und damit einer vorzeitigen Verletzung des Gewebes bestehe.

Nach dem oben beschriebenen Betätigen der Schneidklinge und Abtrennen des Gewebes wird die Schneidklinge in ihrer Raststellung zurückgeführt und dann die Klemme geöffnet. Damit gelangt die Schneidklinge wieder in ihre geschützte unwirksame Stellung unter der Schulter 18. Die Klemme kann dann über einen nächsten zu klemmenden und durchzutrennenden Gewebeabschnitt vorgeschoben und wieder geschlossen werden, worauf sich die beschriebenen Vorgänge wiederholen. Es kann in dieser Weise abschnittweise sauber und sicher gearbeitet werden, wobei die Position des Trennschnittes bezüglich des geklemmten Gewebeabschnittes in Längs- und Querrichtung stets genau bestimmt ist.

Es sind Ausführungsvarianten möglich. Die Rastvorrichtung für die Schneidklinge und deren Eingreifen unter die Schulter 18 der Schutzrampe 19 stellt eigentlich eine doppelte Sicherheit gegen ungewollte vorzeitige Bewegungen der Schneidklinge dar. Man könnte gegebenenfalls auf die Schulter 18 verzichten, wenn nur die Schnittkante 10 der Schneidklinge bei der Raststellung derselben an einer Seitenflanke des Kastens der unteren Klemmbacke anliegt, also nirgends frei liegt. Es könnten gegebenenfalls beide Klemmbacken mit Klemmplatten aus hartem Material bestückt sein. Fig. 2 zeigt eine typische Krümmung der Klemmbacken und der Schneidklinge wie sie für Operationen am Parametrium geeignet ist. Die Schneidklinge liegt dabei bezogen auf die Krümmung innen. Für andere Operationen wären gegebenenfalls nicht oder anders gekrümmte Klemmbacken und entsprechend geformte Schneidklingen vorteilhaft.

## Patentansprüche

1. Parametriumschneidklemme mit Klemmbacken (1, 2) und einer Verriegelungsvorrichtung (8) zum Arretieren der Klemmbacken in Klemmstellung, dadurch gekennzeichnet, dass seitlich der Klemmbacken (1, 2) eine bewegliche Schneidklinge (11) vorgesehen ist, die aus einer unwirksamen Offenstellung auf der Seite der einen Klemmbacke (2) in den Bereich einer Gegenschnittkante (9) an der anderen Klemmbacke (1) bewegbar ist, um geklemmtes Gewebe auf einem Abschnitt der Länge der Klemmbacken seitlich derselben durchzuschneiden.

2. Klemme nach Anspruch 1, dadurch gekennzeichnet, dass die Schnittkante (10) der Schneidklinge (11) bei Offenstellung der Klemmbacken (1, 2) und der Schneidklinge hinter eine Schutzrampe (19) an der erwähnten einen Klemmbacke (2) greift.

3. Klemme nach Anspruch 1 oder 2, dadurch gekennzeichnet, dass die Schneidklinge (11) durch eine Rastvorrichtung (4, 17) in ihrer offenen, unwirksamen Stellung gehalten ist.

4. Klemme nach Anspruch 3, dadurch gekennzeichnet, dass die Rastkraft derart bemessen ist, dass nach dem Auslösen der Rastvorrichtung (4, 17) geklemmtes Gewebe schlagartig durchschnitten wird.

5. Klemme nach irgendeinem der Ansprüche 2-4, dadurch gekennzeichnet, dass die erwähnte andere Klemmbacke (1) beim Schliessen der Klemmbacken (1, 2) die Schneidklinge (11) aus dem Bereich der Schutzrampe (19) drängt.

6. Klemme nach irgendeinem der Ansprüche 1-5, dadurch gekennzeichnet, dass die Klemmbacken (1, 2) gekrümmt sind und die Schneidklinge (11) bezogen auf die Krümmung innen liegt.

## Claims

1. A parametric clamping and cutting device with clamping jaws (1, 2) and a locking device (8) for blocking the clamping jaws in a clamping position, characterized in that there is provided at the side of the clamping jaws (1, 2) a mobile cutting blade (11) which is movable from an open, ineffective position at the side of one of the clamping jaws (2) into the range of a countercutting edge (9) at the other clamping jaw (1) for cutting clotted tissue on a section of the length of the clamping jaws, at the side thereof.

2. A device according to claim 1, characterized in that in the open position of the clamping jaws (1, 2) and of the cutting blade (11), the cutting edge (10) of the cutting blade (11) engages behind a protective ramp (19) of the clamping jaw (2) mentioned as one of the clamping jaws.

3. A device according to claim 1 or 2, characterized in that the cutting blade (11) is maintained in its open, ineffective position by a locking device (4, 17).

4. A device according to claim 3, characterized in that the locking force is of such a value that after the release of the locking device (4, 17), clotted tissue is cut suddenly.

5. A device according to anyone of the claims 2-4, characterized in that, when closing the clamping jaws (1, 2), said other clamping jaw (1) pushes the cutting blade (11) out of the protective ramp (19).

6. A device according to anyone of the claims 1-5, characterized in that the clamping jaws (1, 2) are curved and in that the cutting blade (11) is inside with respect to the curvature.

## Revendications

1. Appareil pour pincer et couper le parametrium avec mâchoires de serrage (1, 2) et un dispositif de verrouillage (8) pour bloquer les mâchoires en position de serrage, caractérisé en ce qu'il est prévu sur le côté des mâchoires de serrage (1, 2) une lame tranchante (11) mobile qui est déplaçable d'une position ouverte inactive sur le côté d'une des mâchoires de serrage (2) dans le domaine d'une contre-arête de coupe (9) de l'autre mâchoire de serrage (1) pour sectionner du tissu collé sur une section de la longueur des mâchoires de serrage, à côté de celles-ci.

2. Appareil selon la revendication 1, caractérisé en ce que dans la position ouverte des mâchoires de serrage (1, 2) et de la lame tranchante (11), l'arête coupante (10) de la lame tranchante (11) s'engage derrière une rampe de protection (19) de la mâchoire (2) mentionnée comme l'une des mâchoires de serrage.

3. Appareil selon la revendication 1 ou 2, caractérisé en ce que la lame coupante (11) est maintenue dans sa position ouverte inactive par un dispositif de verrouillage (4, 17).

4. Appareil selon la revendication 3, caractérisé en ce que la force de verrouillage est de valeur telle qu'après le déclenchement du dispositif de verrouillage (4, 17), du tissu collé est coupé brusquement.

5. Appareil selon l'une quelconque des revendications 2-4, caractérisé en ce que ladite autre mâchoire de serrage (1) force la lame coupante (11) hors de la rampe de protection (19), lors de la fermeture des mâchoires de serrage (1, 2).

6. Appareil selon l'une quelconque des revendications 1-5, caractérisé en ce que les mâchoires de serrage (1, 2) sont courbées et en ce que la lame coupante (11) se trouve à l'intérieur par rapport à la courbure.

# FIG.1

IV

1  10  4  13  14  III  16  12.a  15  17  III  8  7

2  19  18  9  11  5  12  3  6

IV

0 134 750

# FIG.2

1  4  14

10  11  5  12  13  7

FIG.3    FIG.4

0 134 750